# EUROPEAN PATENT SPECIFICATION

(11) **EP 0 570 087 B1**
(45) Date of publication and mention of the grant of the patent: **02.08.1995**
(21) Application number: 93250126.5
(22) Date of filing: 05.05.1993
(51) Int. Cl.: A61B 17/064

(54) **Clip for surgical purposes**
Klammer für chirurgische Zwecke
Pince pour utilisation chirurgicale

(30) Priority: 11.05.1992 DE 4215449
(43) Date of publication of application: 18.11.1993
(73) Proprietor: ETHICON INC., Somerville, New Jersey 08876-0151 (US)
(72) Inventor: Schulze, Dale, W-2000 Hamburg 52 (DE)
(74) Representative: UEXKÜLL & STOLBERG

(56) References cited:
- EP-A- 0 085 930
- EP-A- 0 337 874
- WO-A-89/04146
- US-A- 3 958 576

## Description

The invention relates to a clip for surgical purposes, especially for the bringing together of bundles of vessels and tissue structures under endoscopic operation conditions, said clip being made from bendable wire-like material and having a base and limbs projecting from each end thereof, said base being shaped bent backwards opposite to the direction of the limbs.

Mechanical wound closures by means of clips are used in practically all surgical areas in order to bind tissue and hold it together, for example during anastomoses in the whole digestive tract, from the oesophagus to the rectum, and generally when setting clip sutures. The clips applied in such cases are made from bendable wire-like material and are bent into a closed form, which firmly encloses the tissue lying in between, in the area of application by using a clip-setter. Such clips and clip-setters are known for example from the complete Ethicon catalogue published in 1989, pages 119 to 127.

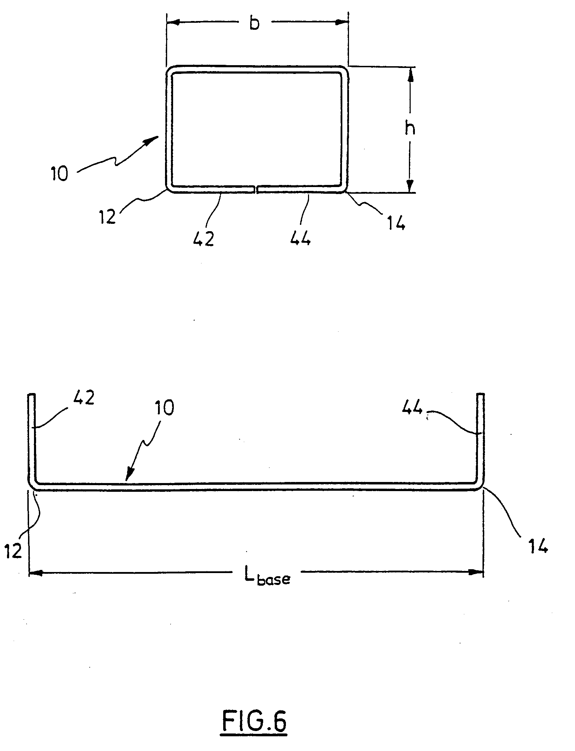

The clips are kept ready in the setters in a form which largely corresponds to that known from office staples. The lower part of Figure 6 shows a correspondingly formed conventional clip. It has a rectilinear bottom side or base 10, from the two ends 12 and 14 of which extend limbs 42 and 44, projecting essentially at right angles. In use, the clip is bent into the closed shape shown in the upper part of Figure 6 with the help of the setter and wraps round the tissue lying in between. The closed clip is essentially rectangular in shape, the limbs essentially forming the upper side of the rectangle and the former base becoming the bottom and the two sides of the rectangle.

Surgical clips are also used in modern minimally invasive operation techniques. In these operations, mostly called endoscopic or laparoscopic, the instruments needed for operative measures are introduced into the body through one or more trocar cannulae to the operation area, for example into the abdominal cavity, and controlled there under endoscopic monitoring. The trocar cannulae have a small diameter in order that only minimal wounds occur when the trocar cannula is introduced through the abdominal wall; the two sizes most often used by surgeons allow passage of 5 mm and 10 mm instruments. Clip setters which are to be used endoscopically must likewise be introduced into the operation area through such trocar cannulae, the clips contained in the setter lying with their rectilinear bottom sides perpendicular to the longitudinal axis of the trocar cannula in the case of known setters and being pushed forward in this position with the setter. This produces the condition that the length of the base must always be less than the internal diameter of the trocar cannula. Since, for medical reasons, the trocar diameters are limited and there are pre-set standardizations for 5 mm and 10 mm trocars, the problem to date has been that only clips with a relatively small base length and thus with a small perimeter and small overall size can be used in endoscopic operations through trocar cannulae.

The lower part of Figure 6 shows a conventional clip prior to setting. It has a straight bottom side or base 10 from whose ends 12 and 14 limbs 42 and 44 project respectively. It is easy to see that much of the perimeter of the finally formed, essentially rectangular clip in the upper part of Figure 6 comes from the originally straight base 10. The height (h) and width (b) of the closed rectangular clip are approximately linked to the length of the base 10 prior to setting (L_{base}) through the relationship ${\text{b + 2 h = L}}_{\text{base}}$ if one assumes that the bottom side and the two sides of the set clip are formed from the base 10. A clip which has, for example, a width b = 8 mm and a height h = 6 mm after setting would, if formed conventionally, have a base length of some 20 mm. The clip to be introduced with its base transversely through a trocar cannula would thus require a trocar cannula with an internal diameter of more than 20 mm. Trocars of this size are not available as standard and their use would also not be generally desirable as, when they are applied, the advantages of minimally invasive surgery would be largely lost. DE-A-32 04 532 discloses the features of the preamble of claim 1.

From German Patent No. 32 04 532 a surgical clip made from bendable wire-like material is know, in which before setting the base does not run along a straight line but is bent backwards, opposite to the direction of the limbs. In this way the free ends of the limbs and the vertex of the base lie approximately along one line. Because of this shape, before setting the clip is somewhat more compact than the clip described before, which has a straight base. The extension of the clip measured transversely with respect to the limbs is, however, again significantly greater than the extension measured in the direction of the limbs so that introducing such clip would require an unadvantageously large trocar cannula too.

It is thus the object of the invention to provide a surgical clip which is introducible with a setter through a trocar cannula having a pre-set diameter and which surgical clip is bendable into a closed form having a larger perimeter than conventional clips.

This object is achieved thanks to the clip with the features of claim 1. Advantageous versions are given in the dependent claims.

The shaping of the clip according to the invention allows a clip with a much greater base length than is the case with the conventional configuration to be pushed forward through a trocar cannula with a given internal diameter. Clips with a greater periphery, i.e. thicker clips, can thereby be used with the available trocar cannulae in endoscopic operations. The clip is so shaped according to the invention that the conventionally rectilinear bottom side or base is formed bent back on itself, so that its ends touch or at least come close to each other. The limbs project, preferably bent slightly backwards, from the brought-together ends. In this way, the base and the limbs essentially form the shape of the capital Greek letter omega (Ω). The bent-back design of the base essentially reduces the effective lateral extension, critical for transport through the trocar cannula, transversally with respect to the transport direction (trocar axis), so that larger clips are introducible into the operation area for a given trocar diameter.

The invention is described below with reference to the drawings, which show:
- Figure 1: a first embodiment in plan view of a clip formed according to the invention;
- Figure 2: another embodiment of a clip in plan view;
- Figures 3 a) to 3 d):: plan views of chronologically successive forms during setting of the clip;
- Figure 4: a side view of the distal end of a clip setter in partial section;
- Figure 5: a plan view of the clip setter from Figure 4 in partial section; and
- Figure 6: a known office staple.

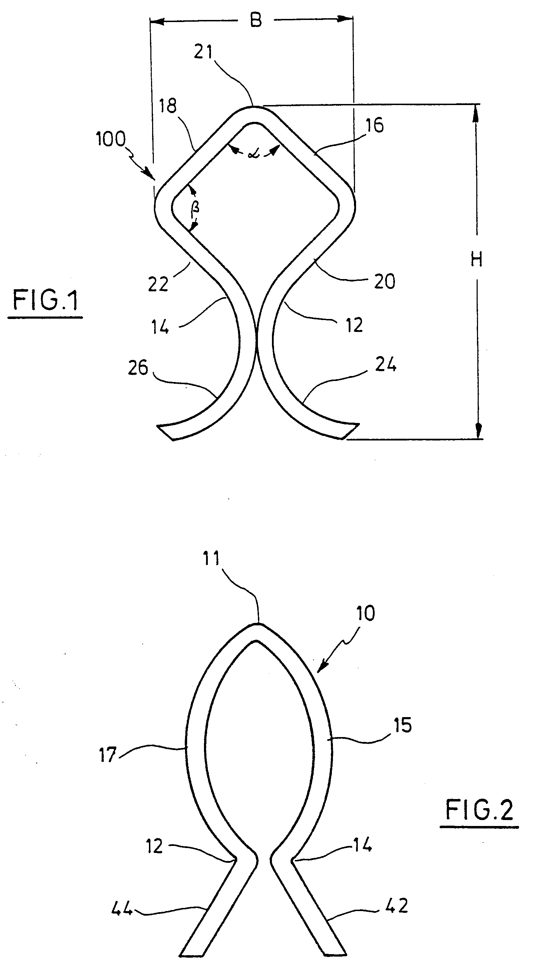

Figures 1 and 2 show embodiments of the clip according to the invention which are introducible through a trocar cannula. The clip shown in Figure 1 obtains its bent-back form through first essentially rectilinear sections 16 and 18 which extend to both sides from a central bend or kink 21 at the top, and second essentially rectilinear sections 20 and 22 attaching respectively thereto. The first and second essentially rectilinear sections 16, 18, 20, 22 together form an essentially closed, rhombus-like contour. In the embodiment represented, the first rectilinear sections 16 and 18 form an angle α, and the first and second rectilinear sections 18 and 22 as well as 16 and 20, respectively, form an angle β, both roughly corresponding to a right angle. The second essentially rectilinear sections 20 and 22 change continuously at their lower end into a curve to which the outwardly curved limbs 24 and 26, respectively, are continuously attached.

The clip represented is symmetrical in relation to a straight line which runs through the tip 21 of the clip and through the contact point of the limbs 24 and 26. The width which is essential for the passage through the trocar cannula, i.e. the lateral extension in relation to the symmetry straight line, is much reduced by the shaping according to the invention compared with conventional clips. A clip which is 8 mm wide and 6 mm high after setting has merely a width B = 6 mm and a height H = 10 mm with the shaping according to the invention, which is to be compared with the base length, i.e. the effective width, of 20 mm in the above example for a conventional clip. This makes the considerable reduction in width, and thus the use of larger clips in the current trocar cannulae, immediately clear. It can also be seen that the reduction in the lateral extension is achieved at the cost of a greater extension in the length or height. The clip is advantageously formed in such a way that the maximum lateral extension in relation to the symmetry straight line in the area of the bent-back base and of the limbs is the same, namely the width in the transitional region between the first and second rectilinear sections 16, 20 and 18, 22 and at the outside tips of the limbs 24, 26.

Figure 2 shows a second embodiment of a clip according to the invention. At its tip, the clip has a bend or kink 11 in the centre of the base, which corresponds to the kink 21 from the embodiment in Figure 1. In principle, the base 10 can also be shaped throughout with continuous curvature, but the configuration with a kink 11 or 21 at the tip is advantageous, as is explained further below. In the embodiment represented, continuously curved sections 15 and 17 extend to both sides from the kink 11, which sections, after exceeding a maximum lateral extension, run together again and touch or come close to each other with their respective end sections 12, 14. In this embodiment, rectilinear limbs 42 and 44 extend from these.

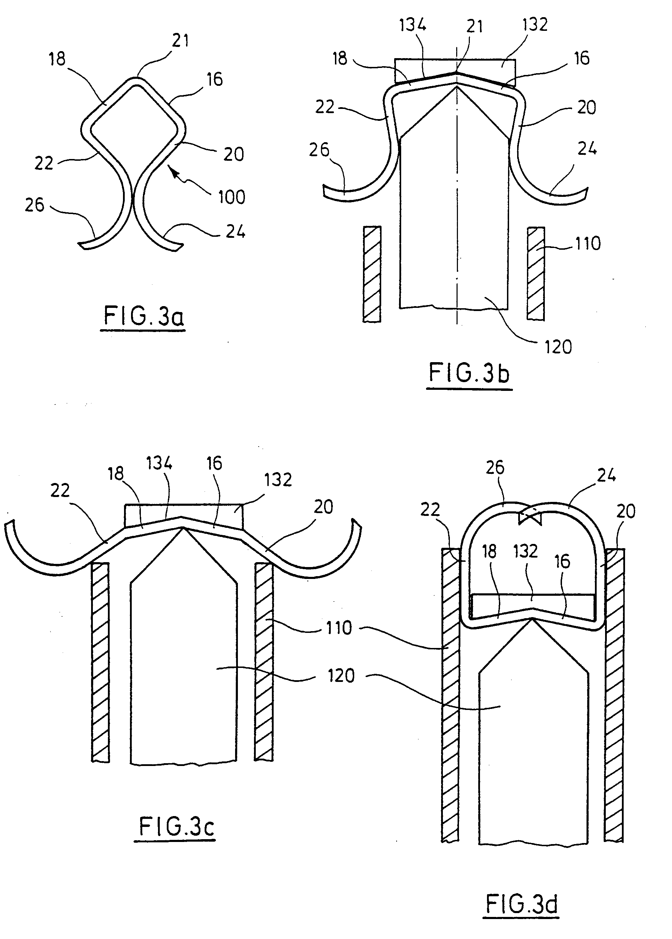

Figures 3 a) to 3 d) display chronologically successive stages in the setting of the clip, in order to illustrate how it functions. Figure 3 a) shows as starting point a clip 100 which largely agrees with the embodiment from Figure 1. The clip 100 is pushed forward through the trocar cannula in a clip setter, as described below, the clips 100 lying arranged one behind the other in a magazine or cartridge. The clips are positioned in a manner so that the tip 21 of one respective clip lies between the limbs 24, 26 of the next clip. Figure 3 b) shows the next stage in the shaping of the clip, after the clip is pushed to the distal tip of the clip setter, which is indicated diagrammatically and described in more detail below. The clip 100 is pushed forward to the tip of the setter and held there by a clamping apparatus comprising an anvil 132 and a movable clamping jaw 120. It will be seen that the first rectilinear sections 16 and 18 lie against the angled front face of the anvil 132. The second rectilinear sections 20 and 22 are already spread apart by a clamping jaw 140 which was inserted from behind between the limbs 24 and 26 and clamps the tip 21 firmly against the anvil 132.

In the next step in Figure 3 c), a sliding sleeve 110 engages from behind at the outward-standing sections 22, 26 and 20, 24 and is pushed further forward against the held clip 100, as a result of which the clip is bent forward around the anvil 132. In this embodiment the first rectilinear sections 16, 18 lie against the anvil 132 and form the bottom side for the clip to be shaped. The second rectilinear sections 20 and 22 are bent forward by sliding sleeve 110 until they adopt the position shown in Figure 3 d). The second rectilinear sections 20, 22 project as sides essentially perpendicularly from the bottom side 16, 18 formed with a slight kink in the centre. At the top side, the clip is closed by the slightly curved limbs 24 and 26. Overall, a shape results which is reminiscent of the capital letter B, a configuration which has proved advantageous in practice. After the clip has been set in the shape shown in Figure 3 d), the tissue lying in between holds safely together.

It can be seen that the configuration of the clip with a kink 11 or 21 in the embodiments of Figures 1 and 2 has, inter alia, the advantage that the clip is more easily positionable in the correct position in front of an anvil 132 with a complementarily angled surface, the kink being pushed into the recess of the angled front surface 134 of the anvil 132.

The clip preferably consists of a wire with a rectangular cross section of 0.5 mm x 1 mm, made from a titanium alloy. Proposed as preferred dimensions for the clip in Figure 3 a) are a width of B = 6 mm and a height of H = 10 mm, which gives a set clip in Figure 3 d) with a width of roughly b = 8 mm and a height of roughly h = 6 mm.

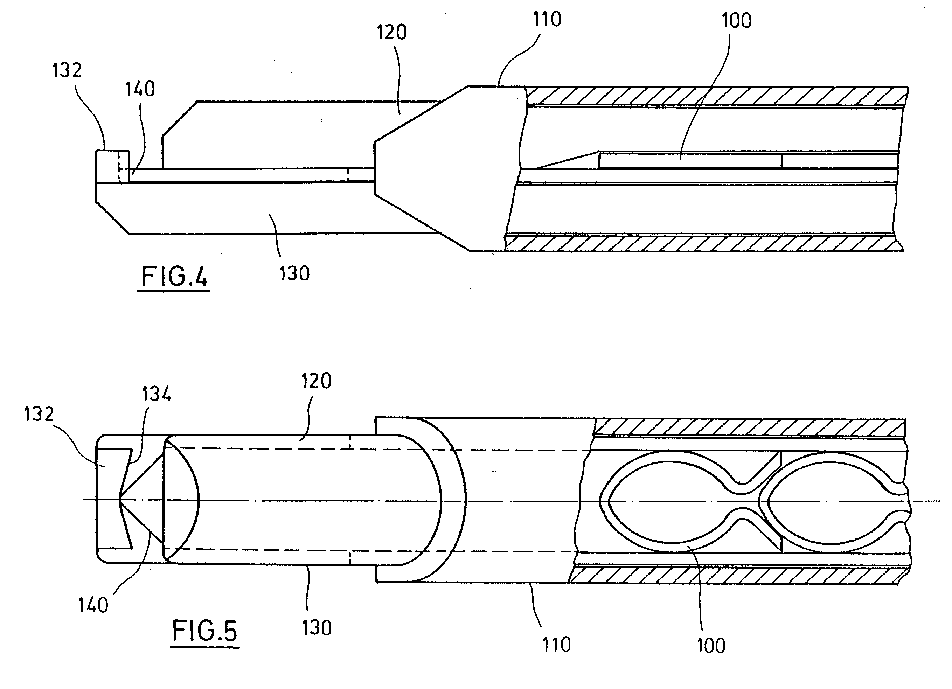

Figures 4 and 5 show a clip setter in which the distal end of the setter to be introduced through the trocar cannula is represented in side view in Figure 4 and in plan view in Figure 5. The setter has an elongated support rod 130 which carries at its distal end a projecting anvil 132 which is designed with a slightly angled front face 134. Seated on the guide rod 130 is a magazine 120 in which a supply of clips 100 is held ready. The clips 100 are arranged lying one behind the other in a row in the magazine 120, so that the tip of each omega-shaped clip 100 lies between the limbs of the clip in front of it. The row of clips is advantageously biased by a pre-stressing force, for example by a spring which presses the clips against each other and forward, so that the respective frontmost clip is brought into the area of the clip-shaping or clip-setting mechanism of the apparatus. Also provided is a clamping jaw 140 which is designed with a front tip in order to engage in the recess of the angled surface 134 of the anvil 132. Clamping jaw 140 is displaceable in longitudinal direction on the guide rod 130 and guided under the clips 100 in the magazine 120, so that when clamping jaw 140 is drawn back the frontmost clip drops into the corresponding space between anvil 132 and clamping jaw 140. Guide rod 130, magazine 120 and clamping jaw 140 are enclosed by a sliding sleeve 110 which is displaceably guided in longitudinal direction.

For actuation, the clip setter has at its proximal end (not shown) an actuation mechanism which can comprise, for example, two oppositely swivellable grips which lie in the user's hand. One of the grips is firmly connected to the guide rod 130 and the other swivellably attached thereat, the swivelling of the second grip transmitting movements onto the clamping jaw 140 and the sliding sleeve 110. To actuate the clip setter, the second grip is swivelled, as a result of which the clamping jaw 140 is drawn off from the anvil 132 so that a free space forms between them. The frontmost clip 100, which is pushed forward by the following clips, slides into this free space, said frontmost clip remaining on the support rod 130 with the tip before the anvil 132. The movement of clamping jaw 140 continues, so that after clip 100 has slid between anvil 132 and clamping jaw 140 the latter is pushed forward again, as a result of which it moves with its tip between the limbs of clip 100 and bends the latter upwards. Delayed against the backward and forward movement of clamping jaw 140, sliding sleeve 110 is pushed forward by the actuation mechanism in order to close the clip in the manner shown in Figure 3 d). Sliding sleeve 110 then returns to its rest position and anvil 132 is likewise drawn back in order to release the set clip.

The clips 100 are aligned in the magazine 120, each kept ready with the tip engaging in the end section of the clip in front of it, a typical magazine size having provision for ten clips. The magazines can easily be made from plastics and insertable into the clip setter. In such a configuration, the setter with the grip and clip-setting mechanism is a reusable instrument into which, for the use in question, a magazine 120 with the clips 100 according to the invention is inserted.

## Claims

1. Clip for surgical purposes, made from bendable wire-like material, which has a base (10) and limbs (42, 44) projecting at its respective ends (12, 14), said base being shaped bent backwards opposite to the direction of the limbs, **characterized in that** the base (10) is shaped bent backwards, starting from its center section, on both sides, so far that the ends (12, 14) of the base touch or come close to each other, base (10) and limbs (42, 44) forming essentially the shape of the capital Greek letter Ω.

2. Clip for surgical purposes according to claim 1, **characterized in that** the shape formed by the base (10) and limbs (42, 44) is essentially symmetrical in relation to a straight line passing through the centre of the base (10) and centrally between its ends (12, 14).

3. Clip for surgical purposes according to claim 2, **characterized in that** the limbs (42, 44) are bent back so far that the clip, in the area of the limbs (42, 44), has - in relation to the symmetry straight line - the same maximum lateral extension as in the region of the bent-back base (10).

4. Clip for surgical purposes according to one of the preceding claims, **characterized in that** the base (10) has a kink (11; 21) in its centre.

5. Clip for surgical purposes according to claim 4, **characterized in that** a respective first, essentially rectilinear section (16, 18) extends from the kink (21) in the centre of the base (10) to each side, said sections forming an essentially right angle.

6. Clip for surgical purposes according to claim 5, **characterized in that** in each case the first essentially rectilinear section (16, 18) passes via an essentially rectilinear kink into a second essentially rectilinear section (20, 22), as a result of which the second essentially rectilinear sections run onto each other.

7. Clip for surgical purposes according to one of the preceding claims, **characterized in that** the limbs (24, 26) are shaped with a curvature which is opposed to the curvature of the base (10).

8. Clip for surgical purposes according to one of the preceding claims, **characterized in that** the clip is made from a titanium alloy or stainless steel.

## Patentansprüche

1. Klammer für chirurgische Zwecke aus verbiegbarem drahtartigem Material, die eine Basis (10) und an deren jeweiligen Enden (12, 14) abstehende Schenkel (42, 44) aufweist, wobei die Basis entgegen der Richtung der Schenkel zurückgebogen geformt ist, **dadurch gekennzeichnet**, daß die Basis (10) ausgehend von ihrem Mittelbereich zu beiden Seiten so weit zurückgebogen geformt ist, daß sich die Enden (12, 14) der Basis berühren oder nahekommen, wobei Basis (10) und Schenkel (42, 44) im wesentlichen die Form des großen griechischen Buchstaben Ω bilden.

2. Klammer für chirurgische Zwecke nach Anspruch 1, **dadurch gekennzeichnet**, daß die durch die Basis (10) und die Schenkel (42, 44) gebildete Form im wesentlichen symmetrisch bezüglich einer durch die Mitte der Basis (10) und mitten zwischen deren Enden (12, 14) verlaufenden Geraden ist.

3. Klammer für chirurgische Zwecke nach Anspruch 2, **dadurch gekennzeichnet**, daß die Schenkel (42, 44) soweit zurückgebogen sind, daß die Klammer im Bereich der Schenkel (42, 44) die - bzgl. der Symmetriegeraden - gleiche maximale seitliche Ausdehnung hat wie Bereich der zurückgebogenen Basis (10).

4. Klammer für chirurgische Zwecke nach einem der vorhergehenden Ansprüche, **dadurch gekennzeichnet**, daß die Basis (10) in ihrer Mitte einen Knick (11; 21) aufweist.

5. Klammer für chirurgische Zwecke nach Anspruch 4, **dadurch gekennzeichnet**, daß von dem Knick (21) in der Mitte der Basis (10) zu jeder Seite jeweils ein erster im wesentlichen geradliniger Bereich (16, 18) ausgeht, wobei die Bereiche einen im wesentlichen rechten Winkel einschließen.

6. Klammer für chirurgische Zwecke nach Anspruch 5, **dadurch gekennzeichnet**, daß jeweils der erste im wesentlichen geradlinige Bereich (16, 18) über einen im wesentlichen rechtwinkligen Knick in einen zweiten im wesentlichen geradlinigen Bereich (20, 22) übergeht, wodurch die zweiten im wesentlichen geradlinigen Bereiche aufeinander zu laufen.

7. Klammer für chirurgische Zwecke nach einem der vorhergehenden Ansprüche, **dadurch gekennzeichnet**, daß die Schenkel (24, 26) mit einer Krümmung geformt sind, die der Krümmung der Basis (10) entgegengesetzt ist.

8. Klammer für chirurgische Zwecke nach einem der vorhergehenden Ansprüche, **dadurch gekennzeichnet**, daß die Klammer aus einer Titanlegierung oder Edelstahl besteht.

## Revendications

1. Agrafe pour utilisation chirurgicale, réalisée à partir d'un matériau en forme de fil pliable, qui a une base (10) et des branches (42, 44) faisant saillie à ses extrémités opposées (12, 14), ladite base étant réalisée pliée vers l'arrière à l'opposé du sens des branches, caractérisée en ce que la base (10) est réalisée pliée vers l'arrière, en partant de sa section centrale, sur les deux côtés, jusqu'à ce que les extrémités (12, 14) de la base se touchent ou viennent à proximité l'une de l'autre, la base (10) et les branches (42, 44) présentant essentiellement la forme de la lettre capitale grecque Ω.

2. Agrafe pour utilisation chirurgicale selon la revendication 1, caractérisée en ce que la forme réalisée par la base (10) et les branches (42, 44) est essentiellement symétrique par rapport à une ligne droite passant par le centre de la base (10) et, de façon centrale, entre ses extrémités (12, 14).

3. Agrafe pour utilisation chirurgicale selon la revendication 2, caractérisée en ce que les branches (42, 44) sont pliées vers l'arrière jusqu'à ce que l'agrafe, dans la zone des branches (42, 44), ait - par rapport à la ligne droite de symétrie - la même dimension latérale maximum que dans la zone de la base pliée vers l'arrière (10).

4. Agrafe pour utilisation chirurgicale selon l'une des revendications précédentes, caractérisée en ce que la base (10) comporte un pli (11; 21) en son centre.

5. Agrafe pour utilisation chirurgicale selon la revendication 4, caractérisée en ce qu'une respective première section, essentiellement rectiligne (16, 18), se prolonge depuis le pli (21) au centre de la base (10), vers chaque côté, lesdites sections formant essentiellement un angle droit.

6. Agrafe pour utilisation chirurgicale selon la revendication 5, caractérisée en ce que, dans chaque cas, la première section essentiellement rectiligne (16, 18) passe, par l'intermédiaire d'un pli essentiellement rectiligne, dans une seconde section essentiellement rectiligne (20, 22), en conséquence de quoi, les secondes sections essentiellement rectilignes sont reliées l'une à l'autre.

7. Agrafe pour utilisation chirurgicale selon l'une des revendications précédentes, caractérisée en ce que les branches (24, 26) sont réalisées avec une courbure qui est opposée à la courbure de la base (10).

8. Agrafe pour utilisation chirurgicale selon l'une des revendications précédentes, caractérisée en ce que l'agrafe est réalisée à base d'un alliage de titane ou d'acier inoxydable.
